# EUROPEAN PATENT APPLICATION

(11) **EP 4 246 453 A1**
(43) Date of publication of application: **20.09.2023**
(21) Application number: 22162492.7
(22) Date of filing: 16.03.2022
(51) Int. Cl.: G06T 19/20

(54) **COMPUTERIZED DENTAL VISUALIZATION**

(71) Applicant: DENTSPLY SIRONA Inc., York, PA 17401 (US); Sirona Dental Systems GmbH, 64625 Bensheim (DE)
(72) Inventor: Adamson, Anders, 64625 Bensheim (DE); Maur, Susanne, 64625 Bensheim (DE); Meyer, Marcel, 64625 Bensheim (DE); Wirjadi, Oliver, 64625 Bensheim (DE)
(74) Representative: Özer, Alpdeniz

(57) **Abstract**

A method for visualizing a dental condition, comprising: providing a plurality of projection images of the dental condition; detecting one or more anatomical features in each of the projection images; rendering, via a textural data-driven logic, texture and/or color information to the one or more anatomical features in the projection images to generate corresponding an at least partially artificially colorized plurality of projection images. The present teachings also relate to a system, an intraoral scanner, software product and a storage medium.

## Description

### TECHNICAL FIELD

The present teachings relate generally to computer-implemented methods and systems for dental scanning, more particularly intraoral scanning and its visualization.

### BACKGROUND

Dental scanners such as intraoral dental scanners, or simply called intraoral scanners, are used for capturing digital dental impressions of a patient. An impression is usually in the form of a three-dimensional ("3D") model and may relate to features of interest of oral anatomy, such as teeth and/or soft and/or hard tissues in the patient's oral cavity. The impression may be digitally stored and/or transmitted to a dental practitioner. The digital impression can also be used for other purposes, such as producing a dental restoration and planning or performing a dental treatment.

It is known to use intraoral scanners based on structured light principle. Such scanners comprise means for projecting light patterns on the intraoral geometry. The pattern when projected on intraoral geometry appears contoured dependent upon the shape of the geometry. The scanners also comprise a camera for capturing light reflected from the geometries which comprises information related to the contours formed in the projected pattern. Signal from the camera is then sent to a computing unit for extracting shape information of the intraoral geometry. Subsequently, a digital impression or 3D model of the intraoral geometry can be reconstructed from the shape information, which may be in the form of a point cloud.

The light patterns used in structured light imaging may be of different types, for example, stripes or dots. Additionally, the patterns may comprise different color components, for example stripes or dots of different colors. A main purpose of structured light imaging is to get as accurate digital impression as possible. Different kinds of patterns and colors can provide different advantages in achieving so.

While intraoral scanning is being performed, the user or practitioner performing it, or even the patient, may like to follow a live visual of the dental condition or the area of the geometry that is being scanned. Thus, a live picture or video of the area in front of the scanning tip of the scanner is helpful for the user and/or the patient. Thus, many structured light scanners provide a visual picturing mode. In the visual picturing mode, the structured light pattern is switched off and a normal light source is switched on to capture a visual picture of the scanning area. In some cases, even a separate camera for visual imaging may be provided, although the same camera may be usable in both the structured light imaging mode and the visual imaging mode. In either case, this necessitates interleaving the structured light imaging mode with the visual imaging mode which can at least slow down the scanning operation. The generation of a digital impression can thus take longer time than necessary. Moreover, additional hardware such as switching unit and light source may be necessary for providing visual imaging mode in addition to the structured light imaging mode, where usually only the latter is used for obtaining the digital impression.

In recent times, self-scanning or self-use type dental scanners have also been introduced. Such scanners can allow a patient to scan their own oral cavity, or in general allow non-practitioners to perform a scanning operation. This can save costs for the user as compared to having a dental practitioner or professional perform the scan and/or dental health monitoring can be improved by performing more regular and/or faster scans at home. For direct to customer market, lower cost solutions are generally more attractive.

There is thus recognized a need for improved intraoral scanning methods and devices.

### SUMMARY

At least some of the limitations associated with the foregoing can be overcome by the subject matter of the accompanying independent claims. At least some of the additional advantageous alternatives will be outlined in the dependent claims.

The applicant has realized by inventive effort that data-driven techniques can be leveraged to create an artificially colorized visual image from the structured light image data captured via an intraoral scanner. A need of a visual imaging mode can thus be obviated. This can save costs as well as make intraoral scanning faster while keeping the scanning process visually followable for the user and/or the patient. The scanning process can thus be kept intuitive despite removal of a separate visual imaging mode.

More specifically, when viewed from a first perspective, there can be provided a computer-implemented method for visualizing a dental condition, which method comprises:
- providing, via an intraoral scanner, a plurality of projection images of the dental condition;
- detecting one or more anatomical features in each of the projection images;
- rendering, via a textural data-driven logic, texture and/or color information to the one or more anatomical features in the projection images to generate an at least partially artificially colorized plurality of projection images.

The at least some of the artificially colorized projection images may be provided for example at an output interface. The output interface may connect to a human machine interface.

The method may also comprise:
- displaying, via a human machine interface ("HMI") unit, at least some of the artificially colorized projection images.

Accordingly, the texture and/or color information is provided automatically to image data received from the intraoral scanner. This can have an advantage that at least a visual imaging mode is prevented from consuming additional time which would otherwise be required for completing a digital impression of the dental condition. Scanning operation can thus be speeded up. Moreover, cost of the intraoral scanner can be reduced while maintaining the same or similar advantage of a visual imaging mode for the user and/or the patient. Hence, dependent upon the application, it may be possible to acquire more structured light images, and/or relax specifications of scanner device, and/or enable faster maneuvering of the scanner during the scan.

Preferably, the displaying operation of the artificially colorized projection images is performed in a real-time or near real-time manner to the capturing of the projection images to provide a better user experience. The term "real-time" is well known to those skilled in the art. Real-time processing or computational operations are those which provide response times within a specified time. The specified time in this context does not require a precise definition as it depends upon the application at hand. Usually, however, in modern computer-implemented processes, the time-period for providing a response to a given input or stimulus is at most a few seconds or below. Preferably, the time-period is in sub-second range, more preferably in milliseconds or microseconds or even lower where it is technically possible. Generally, in computational domain, real-time refers to those operations for which are associated with no perceivable delay. Those skilled in the art shall appreciate that a more precise definition or a specific value is not required for the scope or generality of the present teachings. The rendering operation may thus be performed such that it allows smooth visualization in sync with the speed with which a realistic intraoral scan is performed. The projection images may be provided directly or indirectly via the intraoral scanner. For example, in some cases some or all of the projection images may be processed after being captured by the intraoral scanner. Hence, some or all of the projection images may be directly captured via the intraoral scanner, or they may be processed after being captured by the intraoral scanner. The projection images may be provided at a computer memory or one or more memory storage units. In some cases, the computer memory and/or memory storage may be part of a cloud storage or cloud computing service.

"Projection image" may refer to a structured light image captured via the intraoral scanner. Generally, a plurality of structured light images is provided as image data generated by the intraoral scanner during a scanning operation.

In some cases, the projection images may be in the form of a data-stream, or they may be comprised in a data-stream. The data-stream may at least partially be generated via the intraoral scanner.

"Intraoral scanner" or "intraoral dental scanner" refers to a device that is used to capture a digital impression of oral anatomy or dental condition. The terms may be used interchangeably and refer to a dental scanner that is usable for generating dental impressions for a patient. Thus, the intraoral scanner is usable for generating a digital model of the patient's mouth. The intraoral scanner of the present disclosure is more specifically a device which relies at least on structured light imaging for providing a plurality of projection images. The intraoral scanner thus comprises at least one structured light source and an image sensor for recording reflection of the structured light which is reflected from the oral anatomy of the patient. Output of the image sensor or camera generated in response to the reflections is thus used for providing the projection images. The output may be in the form of a data stream generated continuously or intermittently as the intraoral scanner transmits structured light and records reflections thereof from the oral anatomy of the patient.

It shall be clear that dental condition or oral anatomy refers to anatomical parts in an oral cavity. Oral anatomy may thus include any one or more of teeth and/or soft and/or hard tissue. In some cases, oral anatomy may also include other artificial parts, e.g., replacements or restorations such as any one or more of, dental filling, crown, bridge, braces, veneer, implant, etc. The intraoral scanner records the digital impression of the dental condition by detecting features, especially geometrical features, of the oral anatomy. These intraoral features are then recorded in the digital impression.

"Dental condition" refers more specifically to a state of the oral anatomy at the time at which the plurality of projection images is captured, or the time at which the intraoral scan is performed for providing the projection images.

"Digital impression" refers to a 3D digital model obtained via intraoral scanning of the patient's oral cavity. The digital model may include representation of the patient's teeth and/or hard and/or soft tissues in and around the patient's gums. The digital impression may be usable for providing for the patient any one or more of: dental treatment or procedure, surgical templates, custom devices, prosthetic restorations, and orthodontic aligners.

"Intraoral features" refers to information related to intraoral structures such as dentition and/or gingiva and/or other intraoral anatomical features of oral anatomy of a patient. Alternatively, or in addition, at least some of the intraoral structures of which intraoral features are recorded may be one or more artificial structures such as one or more dental replacements, e.g., dental crown, bridge, veneer, etc. Alternatively, or in addition, the intraoral structure may in some cases even be a scan body (or scanbody) or other natural and/or artificial structure. The intraoral features, more importantly surface data are recorded for building a digital impression of a patient's oral anatomy. Usually for producing the digital impression, structures which are rigidly attached to the jaw of the patient are of more interest.

"Anatomical feature" refers to information related to a specific oral anatomy of the patient. For example, an anatomical feature may be information related to a specific tooth or a group of teeth. Thus, anatomical features may even refer to information related to any one or more intraoral structures such as, dentition, gingiva, nerve channel, extraction site, jaw bone, and condyle. Alternatively, or in addition, anatomical features may be one or more artificial structures such as one or more dental replacements, e.g., dental crown, braces, veneer, bridge. Alternatively, or in addition, an anatomical feature may in some cases even be a scan body (or scanbody) or other natural and/or artificial structure attached to the jaw of the patient. Alternatively, or additionally, the anatomical feature may even be information related to a pathology or condition. As non-limiting examples, pathology or condition may be any one or more of, fracture of tooth or bone, caries, radiolucency, impaction, or any other characterizable state of the oral anatomy or any part thereof. A few non-limiting examples of the anatomical features are, any one or more of: shape, outline or boundary of any one or a group of teeth, their arrangement, sequence in any direction, a presence or a lack of presence of an expected feature such as a given tooth type. Alternatively, or additionally, the anatomical features may also be a shape, region, or a spot detected within an area or interest, such as an outline or boundary. Alternatively, or additionally, the anatomical features may also be, or they may be detected via, an interrelation between two different anatomical features. For example, a boundary between a tooth and gingiva.

Preferably, detection of the anatomical features is performed using a data-driven approach. Hence, the detection of anatomical features may be performed using an anatomy data-driven logic, which may either refer to a single logic providing detection for multiple anatomical features, or it may collectively refer to a group of separate data-driven logics at least one of which logics is used for detecting a single anatomical feature and/or at least one of which logics is used for detecting a group of anatomical features.

"Structured light" in context of "structured light imaging" refers to projected light which comprises a pre-determined pattern. The structured light or pattern is projected onto a target surface for determining topography of the surface. The surface topography induces distortion in the pattern which is projected onto it, The distortion is recorded via an imaging sensor or camera and further analyzed to extract surface information and eventually build a digital model of the surface. It shall be appreciated that in context of intraoral scanning, the target surface is usually oral anatomy of a patient. As the intraoral scanner is moved along the areas or locations adjacent to the surface, the digital impression of the oral anatomy is expanded by stitching together the surface data from different locations. The term "structured light imaging" thus refers to the process of projecting structured light patterns and extracting the surface data from the reflections of said patterns.

"Visual imaging" refers to the process of producing a visual image or photographic image of an object. More specifically, visual imaging refers to imaging which produces an image which shows natural or near natural colors and/or texture of the object which is imaged. Accordingly, visual imaging may be performed using a white light or a light which allows the resulting visual image to show natural or near natural colors and/or texture to a human. Hence, when a visual image is shown via the HMI, the user may be provided a naturalistic reproduction of the object, at least in terms of color and/or texture. It shall be understood that for a greyscale visual image, texture is a more central feature, whereas, for a color image, at least colors should be naturalistic, preferably both color and texture.

"Human machine interface" or "HMI" refers to a device or system which comprises at least one video unit or display screen for displaying to a user at least the artificially colorized projection image.

Thus, the HMI system may comprise a visual display or screen for displaying visual images or video. Optionally, the HMI system may also comprise an audio device. For example, the HMI system may also comprise a loudspeaker for outputting audio. The video unit may comprise a video screen which may be active or passive, for example an LCD screen, OLED screen or projection based video system. Alternatively, or additionally, the video unit may comprise an augmented reality ("AR") device.

The HMI may even comprise one or more input devices for receiving user input.

"Artificially colorized projection image" refers to an image to which color information and/or texture information has been at least artificially provided via a computer logic.

The artificially colorized projection image may either be an artificially colorized and/or texturized structured light image and/or it may be an artificially colorized and/or texturized projection view of a 3D model of the dental condition, which view is viewable on the HMI.

"Textural data-driven logic" refers to a data-driven logic which is used for rendering texture and/or color information to the one or more anatomical features in the projection images. An output of the textural data-driven logic is the at least partially artificially colorized plurality of projection images.

The textural data-driven logic may apply texture and/or color information to any one or more of the detected anatomical features. The detection operation may or may not be done by the textural data-driven logic.

As it shall be discussed further, in some cases, the detection of the anatomical features may be performed via an anatomy data-driven logic. The textural data-driven logic may in such cases be arranged in a serial manner to the anatomy data-driven logic, whereby output of the anatomy data-driven logic is provided directly or indirectly as an input to the textural data-driven logic.

Thus, according to an aspect, the detection of one or more anatomical features in the projection images is performed using an anatomy data-driven logic.

"Anatomy data-driven logic" may refer to a data-driven logic which is trained or pretrained in a supervised manner using anatomy training data comprising projection images with annotation data specifying respective anatomical features. Alternatively, or additionally, the anatomy data-driven logic may be trained in an unsupervised manner.

The anatomy data-driven logic is provided with the projection images as input. The anatomy data-driven logic may provide anatomy data as an output. The anatomy data may thus comprise information related to one or more anatomical features.

According to an aspect, the anatomy data-driven logic performs one or more segmentation operations for detecting the one or more pre-selected anatomical features. Alternatively, or additionally, the anatomy data-driven logic performs detection and/or localization operations for detecting the one or more pre-selected anatomical features.

Thus, in general, the anatomy data-driven logic extracts features of interest, or the pre-selected anatomical features, from the projection images.

In some cases, the anatomy data-driven logic and the textural data-driven logic may be the same logic, for example a combined logic which is a data-driven logic for performing the detection of one or more anatomical features as well as rendering texture and/or color information as appropriate to each of the respective images. Thus, in such a case, the projection images may be provided via an input of the combined logic, and the artificially colorized projection images may be provided via an output of the combined logic.

"Segmentation operation" in the present context refers to a computerized processing operation which concludes with demarcation of at least one anatomical feature with a computer generated feature boundary.

The anatomy data-driven logic may perform a segmentation operation for detecting the one or more pre-selected anatomical features. Thus, the anatomy data-driven logic may segment at least one of the projection images for detection. Thus, the anatomy data-driven logic may extract attributes or one or more objects of interest from at least one of the projection images. The anatomy data-driven logic may classify every pixel in the respective projection image to a class according to its context such that each pixel is assigned to a specific object. A non-limiting example of a segmentation operation when applied using the present teachings may be an outline around a pre-selected anatomical feature, such as a specific tooth or even a group of teeth. Another non-limiting example is an outline around a specific pathology. Said segmented outline corresponds to the shape of the respective anatomical feature as visible in the respective projection image.

There may be a plurality of segmented objects in any given projection image.

In addition to the data-driven approach, the segmentation operation may be supplemented by an analytical model using any suitable segmentation algorithm, e.g., point and line detection, edge linking, and/or thresholding method, histogram, adaptive, and their likes.

A "localization" operation in the present context refers to a computerized processing operation which concludes with a more general region or location within which a specific anatomical feature is located in a projection image. The localization may also result in a boundary within with said anatomical feature is located, however, unlike a segmentation operation, a pixel-by-pixel evaluation is not performed. Hence, a localization boundary, if generated, may be broader than a segmented boundary around a given anatomical feature. Additionally, or alternatively, a localization operation may conclude with annotating or tagging a specific anatomical feature, for example in any location on said anatomical feature.

Similarly, in addition to the data-driven approach, the localization operation may be supplemented by an analytical model using any suitable localization algorithm, e.g., edge and shape detection, and their likes.

"Combined logic" refers to a logic comprising at least one of the two different data-driven logics disclosed herein. The combined logic may be pretrained in a supervised manner using end-to-end training process. The end-to-end training process in this context refers to at least partially annotated data comprising inputs and corresponding outputs. In some cases, the combined logic may be trained fully or partially in an unsupervised manner.

"Data-driven logic" refers to a logic, which at least partially derives its functionality from training data. In contrast to a rigorous or analytical logic which is based in programmed instructions for performing a particular logical task, a data-driven logic can allow forming such instructions at least partially automatically using the training data. The use of data-driven logic can allow to describe logical and/or mathematical relationships without solving equations. This can reduce computational power and/or improve speed. Moreover, data-driven logic may be able to detect patterns or indications (e.g., in input or input data provided to the data-driven logic) which can otherwise not be known or may be difficult to implement as an analytical logic form.

The data-driven logic may be in software and/or hardware form, for example, executable via one or more computing units.

It shall be appreciated that in the present context, the data-driven logic refers to a trained mathematical logic which is parametrized according to the respective training data set. For example, the anatomy data-driven logic is parameterized via its respective training data to detect one or more anatomical features. An untrained logic lacks this information. Hence, the untrained logic or model is incapable for performing a desired detection. Feature engineering and training with the respective training datasets thus enables parametrization of the untrained logic. The result of such a training phase is the respective data-driven model, which as a result of the training process, preferably solely as a result of the training process, provides interrelations and logical capability related to the purpose for which the respective data-driven logic is to be used.

The data-driven logic preferably comprises, or it is a regression logic. In some cases, the data-driven logic may be combined with or it may further include an analytical logic. The analytical logic may describe the relation between its input and output as a function or one or more mathematical equations or logical criteria. Thus, any of the data-driven logics described herein may even be a hybrid logic. A hybrid logic refers to a logic which comprises first-principles parts, so called white-box described via a set of equations e.g., mathematical equations, as well as data-driven parts as explained previously. The data-driven part may also be called black-box logic or model. Hence, the data-driven logic in some cases may be a combination of white-box logic or analytical logic, and black-box logic. In some cases, the data-driven logic may be, or it may even have a grey-box part. A grey-box logic in this context refers to a logic or model which combines a partial analytical structure with a data-driven part using training data to complete the model.

According to an aspect, the projection images can be used to build a visualized 3D model of the dental condition. During the dental scan, it is often rendered a 3D visualization of the dental condition on the HMI. This visualization can help the dental practitioner and patient know the completion status of the intraoral scan. As the scan progresses, the 3D visualization is incrementally built or completed using data from the projection images. Such a 3D visualization or view can be helpful in guiding the user to show which parts of the oral anatomy have been scanned and which regions are remaining or are incomplete. At the start of the scan, the 3D view may be empty, or it may show an outline of a dental arch. As the intraoral scan progresses, the 3D view is enhanced with dental features rendered to it. To get a better angle to view, the dental practitioner may adjust the projection or viewing angle of the 3D view. Thus, viewing the 3D visualization from different angles and/or lighting conditions may be possible.

It may be advantageous to render photo realistic features to the 3D view or model, preferably with photorealistic lighting which can help the dental practitioner get a more realistic feedback between the scan and the resulting visualization. The applicant has realized that the teachings can not only provide the artificially colorized projection images, but also a visualized 3D model which can be used as said 3D visualization for the dental practitioner and/or the patient. The present teachings can allow providing a more realistic 3D view.

Thus, the method may also comprise:
- building, using at least some of the projection images, a visualized 3D model of the dental condition,
- rendering, via a 3D data-driven logic, texture and/or color information to one or more anatomical features in the visualized 3D model to generate an at least partially artificially colorized 3D model.

The 3D data-driven logic may be a separate data-driven logic, or the textural data-driven logic and the 3D data-driven logic may be in a combined form. It is also contemplated that the anatomy data-driven logic, the textural data-driven logic and the 3D data-driven logic in some cases may be the same combined logic.

Those skilled in the art shall appreciate that the aspect of visualized 3D model is also novel and patentable in its own right. Hence, when viewed from another perspective, there can also be provided a method for visualizing a dental condition, which method comprises:
- providing, via an intraoral scanner, a plurality of projection images of the dental condition;
- building, using at least some of the projection images, a visualized 3D model of the dental condition;
- rendering, via a 3D data-driven logic, texture and/or color information to one or more anatomical features in the visualized 3D model to generate an at least partially artificially colorized 3D model.

The at least partially artificially colorized 3D model is preferably displayed at the same HMI. Thus, the method may comprise:
- displaying, via the human machine interface unit, the at least partially artificially colorized 3D model.

Those skilled in the art shall appreciate that the steps may be repeated as the visualized 3D model is being built. For example, the method may comprise:
- incrementally building the visualized 3D model further in response to further projection images captured via the intraoral scanner,
- incrementally rendering texture and/or color information to correspondingly incrementally built visualized 3D model to further build the at least partially artificially colorized 3D model.

Thus, in response to the change in the scan location of the intraoral scanner, the visualized 3D model is further enriched and the texture and/or color information is automatically rendered accordingly.

According to an aspect, the anatomy data-driven logic is trained using anatomy training data which comprise a plurality of historical projection images which are tagged with at least one anatomical feature.

According to an aspect, the textural data-driven logic is trained using textural training data which comprise a plurality of historical projection images tagged with anatomical data, and their corresponding respective visual images which include texture and/or color information. Ideally, the visual images are the photographic images acquired during the intraoral scan as the respective tagged historical projection images.

According to an aspect, the 3D data-driven logic is trained using 3D training data which comprise a plurality of historical 3D visualizations and their corresponding colorized and/or texturized forms. The historical 3D visualizations may comprise historical visualized 3D models, and optionally also historical projection images.

It shall be appreciated that in cases where any two or more of the data-driven logics are combined, the combined logic will have been trained using the corresponding data. For example, in cases where the combined logic includes the anatomy detection and rendering, the combined training data may comprise a plurality of historical projection images and their associated visual images. The visual images include texture and/or color information. Thus, an end-to-end training can be performed thus enabling the combined logic to provide the artificially colorized projection images by analyzing the projection images.

Similarly, in cases where the combined logic performs anatomy detection, rendering of both the projection images as well as the visualized 3D model, the combined training data may comprise a plurality of historical projection images, their associated visual images, as well as their corresponding colorized 3D models.

According to an aspect, the at least partially artificially colorized 3D model is displayed from a perspective, selectable via a user input. Preferably, the rendering of texture and/or color information to the visualized 3D model is performed dependent upon the perspective view. By doing so, instead of an artificial looking image, a more photorealistic representation can be provided to the user. The 3D data-driven logic or the combined logic can thus adapt the texture and/or color rendering dependent upon the perspective view.

The applicant has also realized that a structured light image already comprises some photographic information. This information can be leveraged to provide or improve quality of the artificially colorized projection image and/or the artificially colorized 3D model. Since the structured light image comprises a known pattern in terms of color and/or profile, one or more image correction operations may be performed on a structured light image to obtain the artificially colorized projection image and/or the artificially colorized 3D model.

Thus, according to another aspect, rendering of the texture and/or color information in the projection images and/or the visualized 3D model involves at least one image correction operation.

The image correction operation may for example include extracting color and/or texture information from certain portions of the projection image. For example, the part or parts of the projection image where the structured light is more neutral can be used to detect or estimate the actual color of the part or parts of the dental condition where the scan is being performed. This can be used to determine how the projection image should be altered to obtain an artificial white light visual image or an approximation thereof. This artificial white light visual image can be used to provide a more realistic artificially colorized projection images and/or artificially colorized 3D model.

Additionally, or alternatively, other operations such as filtering, e.g., color filtering, inversing, averaging, edge detection, or their likes may be performed for detection and subsequently performing the image correction operation. Thus, one or more of the projection images may be analyzed, and dependent upon the analysis one or more image correction operations may be performed, e.g., on some or all projection images, to obtain the artificially colorized projection images and/or the artificially colorized 3D model. As a non-limiting example, the image correction operation may be removing color-coded pattern information from at least one of the projection images. Additionally, or alternatively, the image correction operation may include replacing, substituting, or altering information, for example, substituting a color with another, and/or a texture with another. As another non-limiting example, the image correction operation may involve mixing a color in one or more projection images and/or the visualized 3D model with a preselected color to obtain a corrected color in parts or as a whole in the respective projection images and/or visualized 3D model, to obtain more color corrected results, i.e., at least partially artificially colorized plurality of projection images and/or the at least partially artificially colorized 3D model. Similarly, a texture mixing can also be contemplated as an additional or alternative aspect. In some cases, the image correction operation may be a part of the rendering operation.

It shall be appreciated that the present teachings can be particularly advantageous for intraoral scanners which do not comprise means or functionality for capturing white light visual images. Significant cost savings can thus be obtained by eliminating the associated hardware and/or software. Even in intraoral scanners with capability for capturing white light visual images, a fast mode can be provided to speed up the scan process by preventing a white light capture mode at least partially. A possibility can be to deploy more intelligent solutions to capture white light image only when it is required and provide artificially colorized projection images for the rest. A non-limiting example of triggering a white light image can be cases where the respective data-driven logic lacks capability to provide a photorealistic image, for example, due to unusual parts of dental condition which may not have been parameterized via the training data which were available at the time. Another example can be to calibrate certain parameters such as color tone, texture, etc., for which a quick white light image may be used as a reference for the artificially colorized projection images. Similar reasoning can also apply for the artificially colorized 3D model. As it shall be appreciated, the dental scanning process can be made faster whilst providing more realistic images to the user.

When viewed from yet another perspective, there can also be provided a system comprising means for performing any of the herein disclosed method steps. Or there can be provided a system, wherein the system is configured to perform any of the herein disclosed method steps.

For example, when viewed from another perspective, there may be provided an intraoral scanner or an intraoral scanning system comprising:
- a structured light source for projecting structured light on a dental condition;
- a sensor unit for receiving reflection of the structured light from the intraoral surfaces, and generating a plurality of projection images of the dental condition;
- an interface for connecting to one or more computing units, any of which computing units are configured to:
- detect one or more anatomical features;
- render, via a textural data-driven logic, texture and/or color information to one or more anatomical features in the projection images to generate corresponding at least partially artificially colorized plurality of projection images;
- provide, via an output interface, at least some of the artificially colorized projection images.

The output interface may be used to provide some or all of the artificially colorized projection images to an HMI. In some cases, the interface and the output interface may be the same unit. The interface and/or the output interface may be a hardware unit and/or a software module. Either or both of the interfaces may be in the form of, or they may comprise, an application programming interface ("API").

Any of the systems herein disclosed may comprise one or more computing units. Optionally, any of the systems and/or any of the computing units may be configured to operatively connect to a network, e.g., via one or more network interfaces and/or connectivity interfaces. The sensor unit may be a camera or their likes. In some cases, at least one of the computing unit may be part of a cloud computing service.

In some cases, the intraoral scanner or the intraoral scanning system may comprise the HMI for displaying at least some of the artificially colorized projection images.

It shall also be appreciated that, alternatively or additionally, any of the computing units may also be configured to:
- build, using at least some of the projection images, a visualized 3D model of the dental condition,
- render, via a 3D data-driven logic, texture and/or color information to one or more anatomical features in the visualized 3D model to generate an at least partially artificially colorized 3D model.

Hence, the rendering of the texture and/or color information to generate an at least partially artificially colorized 3D model may be done alternatively or in addition to the rendering of the texture and/or color information to generate corresponding at least a partially artificially colorized plurality of projection images.

When viewed from another perspective, there can also be provided a computer software product, or a non-transitory computer-readable storage medium storing the program, comprising instructions which when executed by a suitable computing unit, or a system comprising the computing unit, cause any one or more of the computing units to perform any of the herein disclosed method steps.

For example, when viewed from a perspective, there can be provided a computer software product, or a non-transitory computer-readable storage medium storing the program, comprising instructions which when executed by a suitable computing unit, or a system comprising the computing unit, cause any of the computing units to:
- detect, in a plurality of projection images of a dental condition, one or more anatomical features in each of the projection images; wherein the projection images have been provided via an intraoral scanner,
- render, via a textural data-driven logic, texture and/or color information to the one or more anatomical features in the projection images to generate corresponding an at least partially artificially colorized plurality of projection images.

The software product, or the non-transitory computer-readable storage medium storing the program, may also comprise instructions, which cause any of the computing units to:
- display, via a human machine interface unit, at least some of the artificially colorized projection images.

A computer-readable data medium or carrier includes any suitable data storage device on which one or more sets of instructions (or software) are stored for implementing any one or more methodologies disclosed herein. The instructions may even reside partially, or completely, within the main memory and/or within the processor during execution thereof by the computing unit, and main memory, which may constitute computer-readable storage media. The instructions may even be transmitted or received over a network via a network device.

The computer program for implementing any one or more of the embodiments described herein may be stored and/or distributed on a suitable medium, such as an optical storage medium or a solid-state medium supplied together with or as a part of another hardware, but may also be distributed in other forms, such as via the Internet or other wired or wireless telecommunication systems. However, the computer program may also be presented over a network like the World Wide Web and can be downloaded into the working memory of a data processor from such a network.

Furthermore, a data carrier or a data storage medium for making a computer program product available for downloading can also be provided, which computer program product is arranged to perform a method according to any of the methods herein disclosed.

When viewed from another perspective, there can also be provided a computing unit comprising the computer program code for performing the method herein disclosed. Also, there can be provided a computing unit operatively coupled to a memory storage comprising the computer program code for carrying out any of the methods herein disclosed.

"Computing unit", "computing device", "processing unit" or "processing device" may comprise, or it may be, a processing means or computer processor such as a microprocessor, microcontroller, or the likes, having one or more computer processing cores.

"Computer processor" refers to an arbitrary logic circuitry configured for performing basic operations of a computer or system, and/or, generally, to a device which is configured for performing calculations or logic operations. In particular, the processing means or computer processor may be configured for processing basic instructions that drive the computer or system. As an example, the processing means or computer processor may comprise at least one arithmetic logic unit ("ALU"), at least one floating point unit ("FPU"), such as a math coprocessor or a numeric coprocessor, a plurality of registers, specifically registers configured for supplying operands to the ALU and storing results of operations, and a memory, such as an L1 and L2 cache memory. In particular, the processing means or computer processor may be a multi core processor. Specifically, the processing means or computer processor may be or may comprise a central processing unit ("CPU"). The processing means or computer processor may be a complex instruction set computing ("CISC") microprocessor, reduced instruction set computing microprocessor ("RISC"), Very long instruction word ("VLIW") microprocessor, a processor implementing other instruction sets or processors implementing a combination of instruction sets. The processing means may also be one or more special purpose processing devices such as an application specific integrated circuit ("ASIC"), a field programmable gate array ("FPGA"), a complex programmable logic device ("CPLD"), a digital signal processor ("DSP"), a network processor, or the like. The methods, systems and devices disclosed herein may be implemented as software in a DSP, in a microcontroller, or in any other side processor such as hardware unit within an ASIC, CPLD, or FPGA. It is to be understood that the term processing means or processor may also refer to one or more processing devices, such as a distributed system of processing devices located across multiple computer systems (such as cloud computing), and is not limited to a single device unless otherwise specified.

"Memory storage" may refer to a device for storage of information, in the form of data, in a suitable storage medium. Preferably, the memory storage is a digital storage suitable for storing the information in a digital form which is machine readable, for example digital data that are readable via a computer processor. The memory storage may thus be realized as a digital memory storage device that is readable by a computer processor. Further preferably, the memory storage on the digital memory storage device may also be manipulated by a computer processor. For example, any part of the data recorded on the digital memory storage device may be written and or erased and or overwritten, partially or wholly, with the new data by the computer processor.

"Network" discussed herein may be any suitable kind of data transmission medium, wired, wireless, or their combination. A specific kind of network is not limiting to the scope or generality of the present teachings. The network can hence refer to any suitable arbitrary interconnection between at least one communication end point to another communication end point. Network may comprise one or more distribution points, routers or other types of communication hardware. The interconnection of the network may be formed by means of physically hard wiring, optical and/or wireless radio frequency ("RF") methods. The network specifically may be, or it may comprise, a physical network fully or partially made by hard wiring, such as a fiber optical network or a network fully or partially made by electrically conductive cables or a combination thereof. The network may at least partially comprise the Internet.

"Network interface" refers to a device or a group of one or more hardware and/or software components that allow an operative connection with the network.

"Connectivity interface" or "communication interface" refers to a software and/or hardware interface for establishing communication such as transfer or exchange of signals or data. The communication may either be wired, or it may be wireless. Connectivity interface is preferably based on, or it supports one or more communication protocols. The communication protocol may be a wireless protocol, for example: short distance communication protocol such as Bluetooth^{®}, or Wi-Fi, or long communication protocols such as cellular or mobile network, for example, second generation cellular network ("2G"), 3G, 4G, long term evolution ("LTE"), or 5G. Alternatively, or in addition, the connectivity interface may even be based on proprietary short distance or long distance protocol. The connectivity interface may support any one or more standards and/or proprietary protocols.

That two or more components are "operatively" coupled or connected shall be clear to those skilled in the art. In a non-limiting manner, this means that there may be at least one communicative connection between the coupled or connected components e.g., they are the network interface or any suitable interface. The communicative connection may either be fixed, or it may be removable. Moreover, the communicative connection may either be unidirectional, or it may be bidirectional. Furthermore, the communicative connection may be wired and/or wireless. In some cases, the communicative connection may also be used for providing control signals.

### BRIEF DESCRIPTION OF THE SEVERAL VIEWS OF THE DRAWINGS

Certain aspects of the present teachings will now be discussed with reference to the accompanying drawings that explain said aspects by the way of examples. Since the generality of the present teachings is not dependent on it, the drawings may not be to scale. Method and system aspects may be discussed in conjunction for ease of understanding. Certain features shown in the drawings may be logical features that are shown together with physical features for the sake of understanding and without affecting the generality or scope of the present teachings.
FIG. 1 illustrates a block diagram of a scanning system;
FIG. 2 illustrates a flowchart showing a method aspect of the present teachings.

### DETAILED DESCRIPTION

In accordance with example aspects described herein, methods, systems and computer readable storage media can be provided, e.g., for visualizing a dental condition.

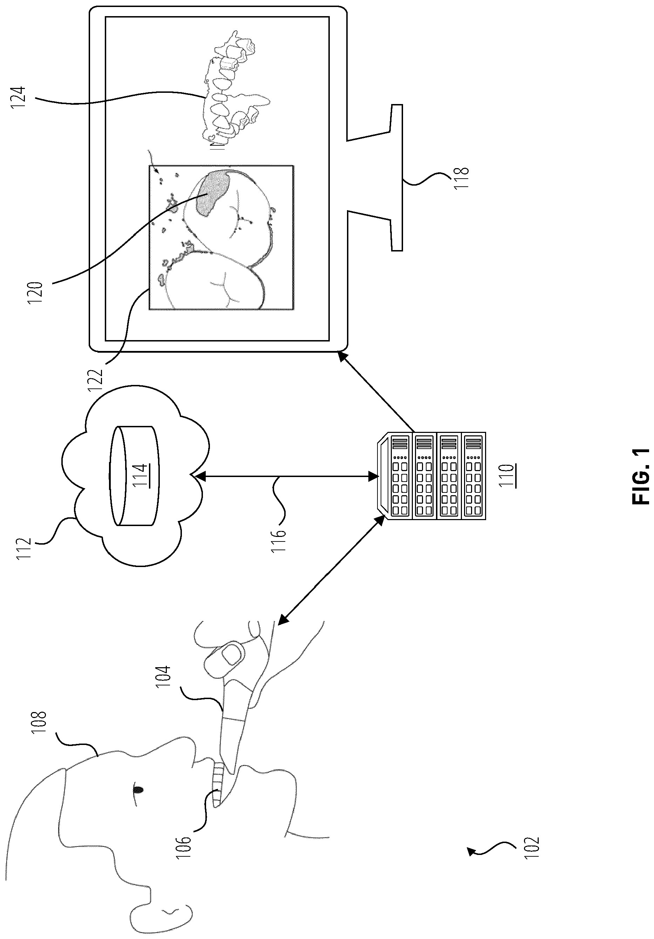

FIG. 1 shows a block diagram 102 of an intraoral scanning system. The system comprises an intraoral scanner 104, which is shown in FIG. 1 being used for scanning dental condition 106 of a patient 108. The intraoral scanner 104 is operatively connected to a computing module 110, which comprises at least one computing unit. In some cases, the computing module 110 may be located at least partially within the intraoral scanner 104. In some cases, the computing module 110 may at least partially be a part of a cloud service 112. Thus, the computing module 110 may be a local device at the location of the intraoral scanner 104 and/or at least partially a remotely located device or system, e.g., one or more cloud services 112 and/or a remote server. The computing module 110 is also operatively connected to a memory storage 114, which may at least partially be a part of the cloud service 112. In this case, the memory storage 114 is shown as a part of the cloud service 112. The intraoral scanner 104 may connect to an external computing module 110 via a connectivity interface (not explicitly shown in FIG. 1). Any local devices, e.g., the intraoral scanner 104 and/or the local computing module 110 may connect to the cloud service 112 via one or more networks 116. A network interface (not explicitly shown in FIG. 1) may be used to connect the local devices to the remote devices, e.g., the cloud service 112. In some cases, the network interface and the connectivity interface may be the same unit.

The computing module 110 is operatively connected to a human machine interface 118 ("HMI"), which in FIG. 1 is shown as a computer screen. The connection between the computing module 110 and the HMI 118 may be via an output interface (not explicitly shown in FIG. 1), which may for example be a computer port or bus, e.g., display port, HDMI, USB, or their like. However, the output interface may even be an API for providing data to one or more computing units on the HMI 118 side. In some cases, the output interface may be a part of the network interface and/or the connectivity interface. Accordingly, it is possible that the three interfaces are the same device.

The intraoral scanner 104 comprises a structured light source (not explicitly shown in FIG. 1) for projecting structured light on the dental condition 106. The intraoral scanner 104 also comprises a sensor unit (not explicitly shown in FIG. 1) for receiving reflection of the structured light from the surface of the dental condition 106. A plurality of projection images of the dental condition 106 is generated in response to receiving the reflection. Thus, during the scan, the intraoral scanner 104 is operated to capture the plurality of projection images of the dental condition 106. The projection images may be in the form of a data stream. These projection images are provided to the computing module 110. The computing module 110 is configured to detect one or more anatomical features in each of the projection images. Advantageously, the detection of one or more anatomical features is performed using an anatomy data-driven logic.

The computing module 110 uses a textural data-driven logic for rendering texture and/or color information 120 to the one or more anatomical features in the projection images to generate corresponding at least partially artificially colorized projection images 122. The texture and/or color information 120 may be added e.g., by editing the projection images and/or by adding one or more layers to the respective projection images. Thus, the at least partially artificially colorized projection images 122 are also preferably in the form of a data stream which is displayed as a video on the HMI 118. Thus, even without capturing a white light image, photorealistic images or video can be provided for the user or patient 108.

Alternatively, or additionally to providing the at least partially artificially colorized projection images 122, an at least partially artificially colorized 3D model 124 can be provided. The HMI 118 can thus show a more photorealistic visual representation of the dental condition 106 as the intraoral scan progresses. The at least partially artificially colorized 3D model 124 is also provided with texture and/or color information which may correspond to the viewing angle or projection view of the model 124 on the screen 118.

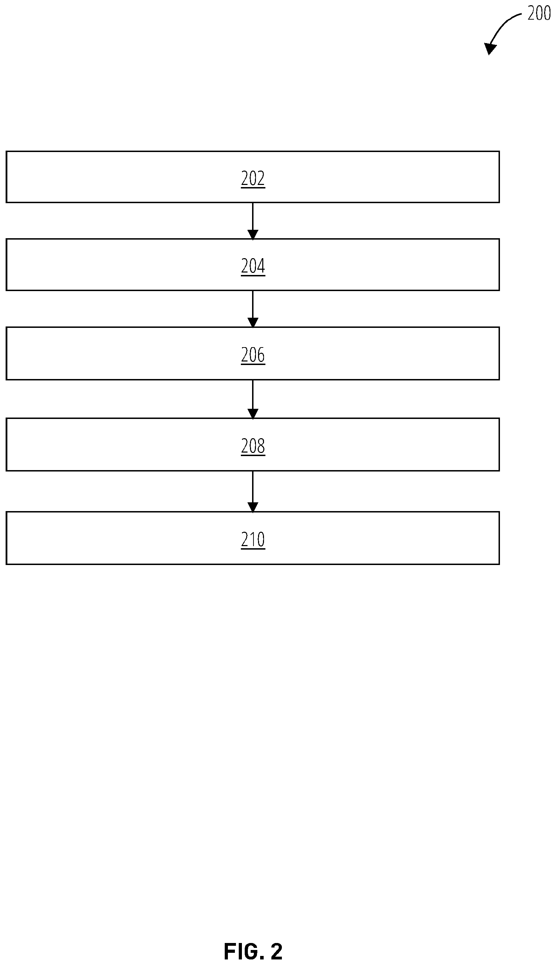

FIG. 2 shows a flowchart 200 which may be implemented as one or more routines which are executed by one or more computing units. At least certain related system aspects shall also be apparent from the following. The flowchart 200 may be implemented as one or more routines in an intraoral scanner and/or an intraoral scanning system.

In block 202, it is provided a plurality of projection images of the dental condition of a patient. The projection images may be provided either directly or indirectly via an intraoral scanner. The intraoral scanner is thus used for providing the projection images related to the dental condition of the patient.

In block 204, it is detected one or more anatomical features in each of the projection images. The dental features may differ from one projection image to another, especially as the projection images change as the scan progresses to different parts of the dental condition. The detection is advantageously performed via an anatomy data-driven logic. In an optional block 206, it is rendered, via a textural data-driven logic, texture and/or color information to the one or more anatomical features in the projection images. Thus, an at least partially artificially colorized plurality of artificially colorized projection image is generated. The textural data-driven logic and the anatomy data-driven logic may be different logics, or they may be the same logic, e.g., a combined logic. Optionally, in block 208, it is built or rendered, using at least some of the projection images, a visualized 3D model of the dental condition. Also optionally, in block 210, it is rendered, via a 3D data-driven logic, texture and/or color information to one or more anatomical features in the visualized 3D model to generate an at least partially artificially colorized 3D model.

It shall be appreciated that the routine may be implemented with block 206 while excluding block 208 and block 210, or they can be combined in any suitable processing order, or the routine may be implemented with block 208 and block 210 while excluding block 206.

In any case, the at least partially artificially colorized plurality of projection images and/or the at least partially artificially colorized 3D model may be provided at an HMI, e.g., via an output interface.

The method steps may be performed in the order as shown listed in the examples or aspects. It should be noted, however, that under specific circumstances a different order may also be possible. Further, it is also possible to perform one or more of the method steps once or repeatedly. These steps may be repeated at regular or irregular time periods. Further, it is possible to perform two or more of the method steps simultaneously or in a timely overlapping fashion, specifically when some or more of the method steps are performed repeatedly. The method may comprise further steps which are not listed.

The word "comprising" does not exclude other elements or steps, and the indefinite article "a" or "an" does not exclude a plurality. A single processing means, processor or controller or other similar unit may fulfill the functions of several items recited in the claims. The mere fact that certain measures are recited in mutually different dependent claims does not indicate that a combination of these measures cannot be used to advantage. Any different signs in the claim should not be construed as limiting the scope.

Further, it should be noted that in the present disclosure, the terms "at least one", "one or more" or similar expressions indicating that a feature or element may be present once or more than once typically may have been used only once when introducing the respective feature or element. Thus, in some cases unless specifically stated otherwise, when referring to the respective feature or element, the expressions "at least one" or "one or more" may not have been repeated, notwithstanding the fact that the respective feature or element may be present once or more than once.

Further, the terms "preferably", "more preferably", "particularly", "more particularly", "specifically", "more specifically" or similar terms are used in conjunction with optional features, without restricting alternative possibilities. Thus, any features introduced by these terms are optional features and are not intended to restrict the scope of the claims in any way. The present teachings may, as those skilled in the art will recognize, be performed by using alternative features. Similarly, the features introduced by "according to an aspect" or similar expressions are intended to be optional features, without any restriction regarding alternatives to the present teachings, without any restrictions regarding the scope of the present teachings and without any restriction regarding the possibility of combining the features introduced in such way with other optional or non-optional features of the present teachings.

Unless otherwise defined, all technical and scientific terms used herein have the same meaning as commonly understood by one of ordinary skill in the art to which the present teachings belong.

Various examples have been disclosed above for a method, a system, a device, a use, software program, and a computing unit comprising the computer program code for carrying out the methods herein disclosed. For example, it has been disclosed a method for visualizing a dental condition, comprising: providing a plurality of projection images of the dental condition; detecting one or more anatomical features in each of the projection images; rendering, via a textural data-driven logic, texture and/or color information to the one or more anatomical features in the projection images to generate corresponding at least partially artificially colorized plurality of projection images. The present teachings also relate to a system, an intraoral scanner, software product and a storage medium. Those skilled in the art will understand however that changes and modifications may be made to those examples without departing from the spirit and scope of the accompanying claims and their equivalence. It will further be appreciated that aspects from the method and product embodiments discussed herein may be freely combined.

Any headings utilized within the description are for convenience only and have no legal or limiting effect.

Summarizing and without excluding further possible embodiments, certain example embodiments of the present teachings are summarized in the following clauses:
Clause 1. A computer-implemented method for visualizing a dental condition, which method comprises:
   - providing, via an intraoral scanner, a plurality of projection images of the dental condition;
   - detecting one or more anatomical features in each of the projection images;
   - rendering, via a textural data-driven logic, texture and/or color information to the one or more anatomical features in the projection images to generate corresponding an at least a partially artificially colorized plurality of projection images.
Clause 2. A computer-implemented method for visualizing a dental condition, which method comprises:
   - providing, via an intraoral scanner, a plurality of projection images of the dental condition;
   - building, using at least some of the projection images, a visualized 3D model of the dental condition;
   - rendering, via a 3D data-driven logic, texture and/or color information to one or more anatomical features in the visualized 3D model to generate an at least partially artificially colorized 3D model.
Clause 3. The method of clause 1, further comprising:
   - building, using at least some of the projection images, a visualized 3D model of the dental condition,
   - rendering, via a 3D data-driven logic, texture and/or color information to one or more anatomical features in the visualized 3D model to generate an at least partially artificially colorized 3D model.
Clause 4. The method of any of the clauses 2 to 3, further comprising:
   - displaying, via a human machine interface unit, at least some of the artificially colorized projection images and/or the at least partially artificially colorized 3D model.
Clause 5. The method of any of the clauses 2 to 4, further comprising:
   - incrementally building the visualized 3D model further in response to further projection images captured via the intraoral scanner,
   - incrementally rendering texture and/or color information to correspondingly incrementally built visualized 3D model to further build the at least partially artificially colorized 3D model.
Clause 6. The method of any one of clauses 1 to 5, wherein the data-driven logic is trained with training data which comprise a plurality of historical projection images and their associated visual images.
Clause 7. The method of any one of clauses 1 to 6, wherein the at least partially artificially colorized 3D model is displayed from a perspective.
Clause 8. The method of clause 7, wherein the rendering of texture and/or color information in the visualized 3D model is performed dependent upon the perspective view.
Clause 9. The method of any one of clauses 1 to 8, wherein rendering of the texture and/or color information in the projection images and/or the visualized 3D model involves at least one image correction operation.
Clause 10. The method of any one of clauses 1 to 9, wherein the intraoral scanner does not comprise a visual image camera for capturing real-time visual intraoral images, or the scanner does not provide white light visual images.
Clause 11. The method of any one of clauses 1 to 10, wherein the respective data-driven logic uses a sequence of projection images for performing the rendering of texture and/or color information.
Clause 12. The method of any one of clauses 1 to 11, wherein the intraoral scanner is projecting color-coded pattern type, and wherein the method comprises:
   - removing color-coded pattern information from at least one of the projection images.
Clause 13. A computer software product, or a non-transitory computer-readable storage medium storing the program, comprising instructions which when executed by suitable one or more computing units cause any of the computing unis to perform the steps of any of the above method clauses.
Clause 14. A system comprising means for performing the steps of any of the above method clauses.
Clause 15. An intraoral scanner comprising:
   - a structured light source for projecting structured light on a dental condition;
   - a sensor unit for receiving reflection of the structured light from the intraoral surfaces, and generating a plurality of projection images of the dental condition;
   - an interface for connecting to one or more computing units, any of which computing units are configured to:
   - detect, in the projection images, one or more anatomical features;
   - render, via a textural data-driven logic, texture and/or color information to one or more anatomical features in the projection images to generate an at least partially artificially colorized plurality of projection images;
   - provide, via an output interface, at least some of the artificially colorized projection images.
Clause 16. The intraoral scanner of clause 15, further comprising a human machine unit for displaying at least some of the artificially colorized projection images.
Clause 17. An intraoral scanning system comprising:
   - a structured light source for projecting structured light on a dental condition;
   - a sensor unit for receiving reflection of the structured light from the intraoral surfaces, and generating a plurality of projection images of the dental condition; and
   - one or more computing units, any of which computing units are configured to:
   - detect, in the projection images, one or more anatomical features;
   - render, via a textural data-driven logic, texture and/or color information to one or more anatomical features in the projection images to generate an at least partially artificially colorized plurality of projection images;
      ; and
   - a human machine interface unit for displaying at least some of the artificially colorized projection images.

## Claims

1. A computer-implemented method for visualizing a dental condition, which method comprises:
- providing, via an intraoral scanner, a plurality of projection images of the dental condition;
- detecting one or more anatomical features in each of the projection images;
- rendering, via a textural data-driven logic, texture and/or color information to the one or more anatomical features in the projection images to generate corresponding an at least partially artificially colorized plurality of projection images.

2. The method of claim 1, further comprising:
- building, using at least some of the projection images, a visualized 3D model of the dental condition,
- rendering, via a 3D data-driven logic, texture and/or color information to one or more anatomical features in the visualized 3D model to generate an at least partially artificially colorized 3D model.

3. The method of claim 1 or claim 2, further comprising:
- displaying, via a human machine interface unit, at least some of the partially artificially colorized projection images and/or the at least partially artificially colorized 3D model.

4. The method of claim 2 or claim 3, further comprising:
- incrementally building the visualized 3D model further in response to further projection images captured via the intraoral scanner,
- incrementally rendering texture and/or color information to correspondingly incrementally built visualized 3D model to further build the at least partially artificially colorized 3D model.

5. The method of any one of claims 1 to 4, wherein the at least partially artificially colorized 3D model is displayed from a perspective.

6. The method of claim 5, wherein the rendering of texture and/or color information in the visualized 3D model is performed dependent upon the perspective view.

7. The method of any one of claims 1 to 6, wherein rendering of the texture and/or color information in the projection images and/or the visualized 3D model involves at least one image correction operation.

8. The method of any one of claims 1 to 7, wherein the respective data-driven logic uses a sequence of projection images for performing the rendering of texture and/or color information.

9. The method of any one of claims 1 to 8, wherein the intraoral scanner is projecting color-coded pattern type, and wherein the method comprises:
- removing color-coded pattern information from at least one of the projection images.

10. A computer-implemented method for visualizing a dental condition, which method comprises:
- providing, via an intraoral scanner, a plurality of projection images of the dental condition;
- building, using at least some of the projection images, a visualized 3D model of the dental condition;
- rendering, via a 3D data-driven logic, texture and/or color information to one or more anatomical features in the visualized 3D model to generate an at least partially artificially colorized 3D model.

11. A computer software product, or a non-transitory computer-readable storage medium storing the program, comprising instructions which when executed by suitable one or more computing units cause any of the computing unis to perform the steps of any of the above method claims.

12. A system comprising means for performing the steps of any of the above method claims.

13. An intraoral scanner comprising:
- a structured light source for projecting structured light on a dental condition;
- a sensor unit for receiving reflection of the structured light from the intraoral surfaces, and generating a plurality of projection images of the dental condition;
- an interface for connecting to one or more computing units, any of which computing units are configured to:
- detect, in the projection images, one or more anatomical features;
- render, via a textural data-driven logic, texture and/or color information to one or more anatomical features in the projection images to generate an at least partially artificially colorized plurality of projection images;
- provide, via an output interface, at least some of the artificially colorized projection images.

14. The intraoral scanner of claim 13, further comprising a human machine interface unit for displaying at least some of the artificially colorized projection images.

15. An intraoral scanning system comprising:
- a structured light source for projecting structured light on a dental condition;
- a sensor unit for receiving reflection of the structured light from the intraoral surfaces, and generating a plurality of projection images of the dental condition; and
- one or more computing units, any of which computing units are configured to:
- detect, in the projection images, one or more anatomical features;
- render, via a textural data-driven logic, texture and/or color information to one or more anatomical features in the projection images to generate an at least partially artificially colorized plurality of projection images;
; and
- a human machine interface unit for displaying at least some of the artificially colorized projection images.
